# EUROPEAN PATENT APPLICATION

(11) **EP 2 436 706 A1**
(43) Date of publication of application: **04.04.2012**
(21) Application number: 10780674.7
(22) Date of filing: 25.05.2010
(51) Int. Cl.: C08G 8/02, C08K 3/00, C08K 5/13, C08L 21/00, C07D 311/60

(54) **CONDENSATION PRODUCT OF RESORCIN AND ACETONE**

(30) Priority: 28.05.2009 JP 2009128655; 06.07.2009 JP 2009159564
(71) Applicant: Sumitomo Chemical Company, Limited, Tokyo 104-8260 (JP)
(72) Inventor: TAKEUCHI, Kenichi, Toyonaka-shi Osaka 560-0002 (JP); OZTURK, Orhan, Toyonaka-shi Osaka 561-0802 (JP)
(74) Representative: Vossius & Partner
(86) International application number: PCT/JP2010/059166
(87) International publication number: WO 2010/137720

(57) **Abstract**

A condensation product of resorcin and acetone, wherein the ratio of the area of the first elution peak to the total area of all peaks is 20 to 50% as determined by gel permeation chromatography of which detector is a differential refractive index detector.

## Description

### Field of the Invention

The present invention relates to a condensation product of resorcin and acetone.

### Background of the Invention

In JP Heisei 9-87425 A, a condensation product of resorcin and acetone wherein a temperature of start of melting is 157°C and a temperature of completion of melting is 228°C and a condensation product of resorcin and acetone wherein a temperature of start of melting is 105°C and a temperature of completion of melting is 139°C are described, and a rubber compositions comprising these condensation products and a rubber component are also disclosed therein.

### Disclosure of the Invention

The present invention provides:
<1> A condensation product of resorcin and acetone, wherein the ratio of the area of the first elution peak to the total area of all peaks is 20 to 50% as determined by gel permeation chromatography of which detector is a differential refractive index detector;
<2> The condensation product according to <1>, wherein its melting point is 120°C or less;
<3> A rubber composition comprising the condensation product according to <1> or <2>, a rubber component, a filler and a sulfur component;
<4> A belt comprising a steel cord covered with the rubber composition according to <3>;
<5> A carcass comprising a carcass fiber cord covered with the rubber composition according to <3>;
<6> A captread or undertread comprising the rubber composition according to <3>;
<7> A pneumatic tire produced by using the rubber composition according to <3>.

### Best Modes for Carrying Out the Invention

The condensation product of the present invention is a condensation product of resorcin and acetone, wherein the ratio of the area of the first elution peak to the total area of all peaks is 20 to 50% as determined by gel permeation chromatography of which detector is a differential refractive index detector. The ratio of the area of the first elution peak to the total area of all peaks is preferably 20 to 40%. Gel permeation chromatography is a kind of liquid chromatography, and it is an analytical method wherein molecules of the components of the sample to be tested which is dissolved in a mobile phase is separated based on the differences in size thereof. Generally, smaller molecule enters into pores of a stationary phase and the elution thereof is late, and therefore, molecules are eluted in the order of size from the biggest molecule difficult to enter into pores. Hereinafter, "first elution peak" means a peak having the shortest retention time in all peaks in chromatogram obtained by analyzing with the above-mentioned gel permeation chromatography.

The melting point of the condensation product of the present invention is preferably 120°C or less. Hereinafter, "melting point" means "a temperature of completion of melting" measured according to Japanese Industrial Standards "method of measuring a melting point and a range of melting of a chemical product (JIS K0064)". It is more preferably 110°C or less. The temperature of start of melting of the above-mentioned condensation product is preferably 100°C or less.

Although the condensation product of the present invention can be produced by condensing resorcin with acetone in the presence of an acid catalyst, it is preferably produced by condensing 2,4,4-trimethyl-2',4',7-trihydroxyflavan with acetone in the presence of an acid catalyst, and 2,4,4-trimethyl-2',4',7-trihydroxyflavan is a condensation product of two molecules of resorcin and two molecules of acetone, and a compound having the following formula:

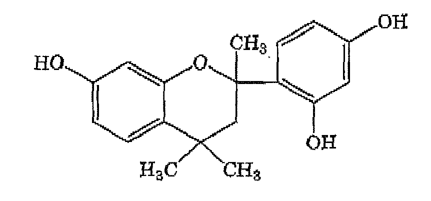

The acid catalyst may be an acid material, and specific examples thereof include sulfuric acid, p-toluenesulfonic acid, hydrochroric acid and phosphoric acid. The acid catalyst may be used as it is, and may be used in a form of an aqueous solution having a suitable concentration. While the used amount of the acid catalyst is not limited, when a condensation reaction of resorcin and acetone is conducted, it is preferably a range of 0.1 to 10 mol% relative to that of resorcin, and more preferably a range of 0.5 to 5 mol%. When a condensation reaction of 2,4,4-trimethyl-2',4',7-trihydroxyflavan and acetone, the used amount of the acid catalyst is preferably a range of 0.2 to 20 mol% relative to that of 2,4,4-trimethyl-2',4','7-trihydroxyflavan, and more preferably a range of 1 to 10 mol%.

While an excess amount of acetone can be used to serve as the solvent, when a condensation reaction of resorcin and acetone is conducted, the used amount of acetone is preferably a range of 2 to 20 moles relative to 1 mole of resorcin. When a condensation reaction of 2,4,4-trimethyl-2',4','7-trihydroxyflavan and acetone, the used amount of the acid catalyst is preferably a range of 2 to 40 moles relative to 1 mole of 2,4, 4-trimethyl-2' , 4',7-trihydroxyflavan.

The condensation reaction may be carried out using acetone as a solvent as described above, and may be conducted in the presence of an organic solvent other than acetone. The reaction is preferably carried out in the presence of an organic solvent other than acetone. Examples of the organic solvent include alcohols, aliphatic hydrocarbons, aromatic hydrocarbons and halogen-substituted aromatic hydrocarbons. Specific examples of the alcohol include methanol and ethanol. Specific examples of the aliphatic hydrocarbon include hexane, heptane, octane and decane. Specific examples of the aromatic hydrocarbon include toluene, xylene and ethylbenzene. Specific examples of the halagen-substitutad aromatic hydrocarbon include chlorobenzene and dichlorobenzene. Among them, preferred is the alcohol, and more preferred is methanol. The used amount of the organic solvent is preferably a range of 1 to 5 parts by weight relative to 1 part by weight of resorcin when a condensation reaction of resorcin and acetone is conducted. When a condensation reaction of 2,4,4-trimethyl-2',4',7-trihydroxyflavan and acetone, the used amount of the organic solvent is preferably a range of 0.5 to 2 parts by weight relative to 1 part by weight of 2,4,4-trimethyl-2',4',7-trihydroxyflavan.

While the reaction temperature is not limited, the reaction is usually carried out in a range of 30°C to a reflux temperature of a reaction mixture. The reaction is conducted with arbitrarily analyzing the reaction mixture with gel permeation chromatography of which detector is a differential refractive index detector and timing wherein the ratio of the area of the first elution peak to the total area of all peaks becomes 20 to 50% in the chromatogram obtained may be set as the reaction end. Usually, the longer the reaction time is, the bigger the ratio of the area of the first elution peak to the total area of all peaks tends to be.

After completion of the reaction, for example, the condensation product can be obtained by neutralizing the reaction mixture obtained with a base, conducting a concentration, extracting the concentrated residu obtained with a water-nonmiscible organic solvent, washing the organic layer obtained, if necessary, with water and conducting a concentration.

The condensation product of the present invention usually contains 2,4,4-trimethyl-2',4',7-trihydroxyflavan, and the content thereof is usually 40% by weight or less.

The condensation product of the present invention can be used as a reinforcing agent for rubbers.

Next, a rubber composition comprising the condensation product of the present invention, a rubber component, a filler and a sulfur component will be illustrated.

Examples of the rubber component include natural rubber, styrene-butadiene copolymerized rubber, butadiene rubber, isoprene rubber and rubber components comprising them as a main component. As the rubber component, commercially available one may be also used, and one produced according to known methods may be also used. The used amount of the condensation product of the present invention is preferably a range of 0.5 to 3 parts by weight relative to 100 parts by weight of the rubber component, and more preferably a range of 1 to 2 parts by weight.

Examples of the fillers include those usually used in the rubber field such as carbon black, silica, talc and clay. Among them, preferred is carbon black, and preferred are HAF (High Abrasion Furnace), SAF (Super Abrasion Furnace) and ISAF (Intermediate SAF). It is effective that some kinds of fillers are used in arbitrarily combination such as a combination of carbon black and silica. While the used amount of the filler is not limited, it is preferably a range of 10 to 100 parts by weight relative to 100 parts by weight of the rubber component, and more preferably a range of 30 to 70 parts by weight.

Examples of the sulfur component include powdery sulfur, precipitated sulfur, colloidal sulfur, insoluble sulfur and highly-dispersive sulfur, and powdery sulfur and insoluble sulfur are preferable. The used amount of sulfur component is preferably a range of 1 to 10 parts by weight relative to 100 parts by weight of the rubber component, and more preferably a range of 2 to 6 parts by weight.

The rubber composition of the present invention can contain a vulcanization accelerator, a methoxylated methylolmelamine resin, an organic cobalt compound and/or zinc oxide.

Examples of the vulcanization accelerator include thiazole-typed vulcanization accelerators, sulfenamide-typed vulcanization accelerators and guanidine-typed vulcanization accelerators described in RUBBER INDUSTRY HANDBOOK, 4th edition published by Society of Rubber Industry, Japan, on Heisei 6, January 20, at pages 412 to 413. When the rubber composition of the present invention contains the vulcanization accelerator, the content of the vulcanization accelerator is preferably a range of 0.5 to 1 part by weight relative to 100 parts by weight of the rubber component, and more preferably a range of 0.6 to 0.8 part by weight.

Examples of the methoxylated methylolmelamine resin include those usually used in the rubber field such as hexakis(methoxymethyl)melamine, pentakis(methoxynethyl)methylolmelamine and tetrakis(methoxymethyl)dimethylolmelamine. Among them, preferred are hexakis(methoxymethyl)melamine and a mixture containing the same as a main component. These methoxylated methylolmelamine resins may be used alone or two or more kinds thereof may be used in combination. When the rubber composition of the present invention contains the methoxylated methylolmelamine resin, the content thereof is preferably a range of about 0.5 to 2 parts by weight per 100 parts of the rubber component, and preferably a range of about 1 to 2 parts by weight.

Examples of the organic cobalt compound include cobalt salts of carboxylic acids such as cobalt naphthenate and cobalt stearate, and an aliphatic acid· boron complex compound (for example, product name "MANOBOND C (registered trade mark)" manufactured by Manchem. Co., Ltd.). When the rubber composition of the present invention contains the organic cobalt compound, the used amount thereof is preferably a range of 0.1 to 0.4 part by weight in terms of cobalt content per 100 parts of the rubber component, and more preferably a range of 0.1 to 0.3 part by weight.

The rubber composition of the present invention may contain one or more kinds of various rubber chemicals usually used in the rubber filed, for example, age resisters such as antioxidants and antiozonants, peptizers, processing aids, waxes, oils, stearic acid and tackifiers, as necessary. While the contents of these rubber chemicals in the rubber composition of the present invention differ depending on the intended use of the rubber composition, each thereof can be used in an amount of the range in which each thereof is usually used in the rubber field.

The rubber composition of the present invention can be led to rubber products via steps such as molding and vulcanization according to methods usually conducted in the rubber field. Especially, it can be used for various tire components such as captread, undertread, belt, carcass, bead, sidewall and rubber chafer. Alternatively, it is also used for antivibration rubbers for cars such as engine mount, strut mount, bush and exhaust hanger, hoses, rubber belts and the like.

The belt can be produced by covering steel cords with the rubber composition of the present invention. The steel cords are usually used in the form of being parallel aligned by pulling. The belt is used, for example, for tires.

It is preferred that steel cords are plated with brass, zinc or alloy containing it and nickel or cobalt from the viewpoint of adhesiveness to rubbers, and those plated with brass are especially preferable. Especially, steel cords plated with brass wherein the content percentage of Cu in the brass-plating is 75% by mass or less, and preferably 55 to 70% by mass are preferable. The twist structure of steel cords is not limited.

The plural belts of the present invention may be layered to be used. The belts of the present invention are mainly used for reinforcing materials of carcass.

Carcass can be produced by conducting extruding processing of the rubber composition of the present invention to fit on the carcass shape followed by applying it up and down carcass fiber cord. The carcass fiber cords are usually used in the form of being parallel aligned by pulling. As carcass fiber cords, polyesters which are good in elastic modulus and resistance to fatigue, are excellent in creep resistance and are inexpensive are preferable. These are used as reinforcing materials for tires in a single or by layering plural thereof.

Captreads or undertread can be also produced by conducting extruding processing of the rubber composition of the present invention.

The pneumatic tire of the present invention is produced by using the rubber composition of the present invention according to the conventional process for producing a pneumatic tire. For example, the extruding processing of the rubber composition of the present invention is conducted to obtain a member for tire and then, it is applied and molded to other tire member or members on a tire molding machine according to conventional methods to be molded to an unvulcanized tire. This unvulcanized tire is heated and pressurized in a vulcanizer to obtain a tire.

### Examples

The present invention will be illustrated in more detail by Examples bellow, but the present invention is not limited to these Example.

### Example 1

Into a 500 mL four-necked flask equipped with a thermometer, a stirrer and a condenser, 80.08 g (0.264 moles) of 2,4,4-trimethyl-2',4',7-trihydroxyflavan (hereinafter, simply referred to as FVR) was added, and the substituting to nitrogen in the flask was conducted. Into the flask, 210.4 g of acetone and 80.0 g of methanol were added, and the mixture obtained was stirred at room temperature to obtain a solution of FVR. To the solution obtained, 0.27 g (0.01 mole) of 98% sulfuric acid was added, and the mixture obtained was heated and refluxed for 5. 5 hours. The reaction mixture obtained was cooled down to room temperature, and then, it was neutralized with 2.12 g of 10% by weight aqueous sodium hydroxide solution. The mixture obtained was concentrated, and the concentrated residue obtained was dissolved in 240 g of ethyl acetate. The solution obtained was washed twice with 100 g of water, and concentrated, and further dried at 80°C for 5 hours under the reduced condition of 1 kPa or less to obtain 65.2 g of a condensation product of resorcin and acetone. The condensation product was analyzed with a permeation chromatography (GPC) area percentage method (its analytical condition was as followed) to find that the ratio of the area of the first elution peak (retention time (RT): 15.5 minutes) to the total area of all peaks was 22.6%. The melting point of the condensation product obtained was measured according to Japanese Industrial Standards "method of measuring a melting point and a range of melting of a chemical product (JIS K0064)" to find that the temperature of start of melting was 93.4°C and the temperature of completion of melting was 100.3°C. Alternatively, the condensation product was analyzed with high-performance liquid chromatography absolute calibration method to find that the content of FVR was 35.7% by weight and the content of resorcin was 0.5% by weight.

### <Analytical condition>

Column: SHODEX KF802 (8 mmφ×30 cm) and SHODEX KF-802.5 (8 mmφ ×30 cm) were joined.
Temperature: 40°C
Mobile phase: tetrahydrofuran
Detector: differential refractive index detector

### Examples 2 to 4 and Comparative Example 1

The condensation product of resorcin and acetone was obtained according to the same manner as that described in Example 1 except that the reflux time was changed to that shown in Table 1. The results are shown in Table 1.

**[Table 1]**

| | | Example 1 | Example 2 | Example 3 | Example 4 | Comparative Example 1 |
|---|---|---|---|---|---|---|
| Reflux Time (Hr) | | 5.5 | 6.5 | 9 | 15 | 4 |
| Yield of Condensation Product (g) | | 65.2 | 71.2 | 85.9 | 88.5 | 78.6 |
| | RT: 15.5 min. | 22.6 | 26.1 | 31.7 | 46.7 | 16.8 |
| Ratio of GPC | RT: 16.2 min. | 33.2 | 34.1 | 34.6 | 32.0 | 31.3 |
| Area (%) | RT: 17.3 min. | 42.7 | 38.2 | 32.5 | 18.6 | 50.5 |
| | RT: 18.5 min. | 1.5 | 1.6 | 1.2 | 2.6 | 1.3 |
| Melting Point of Condensation Product | Temperature of Stare of Melting (°C) | 93.4 | 94.5 | 93.5 | 102.6 | 204.6 |
| | Temperature of Completion of Malting (°C) | 100.3 | 102.6 | 102.9 | 140.2 | 207.3 |
| Content of FVR (% by weight) | | 35.7 | 32.6 | 25.3 | 13.0 | 43.3 |
| Content of Resorcin (% by weight) | | 0.5 | 0.4 | 0.2 | 0.4 | 0.4 |

### Example 5

A 600 mL Laboplastomill manufactured by Toyo Seiki Seisakusho is used as a Banbury mixer and the initial temperature in the system is set at 150°C. One hundred (100) parts by weight of natural rubber (RSS#1), 45 parts by weight of N330 carbon black, 10 parts by weight of hydrous silica (Nipsil AQ manufactured by TOSOH SILICA CORPORATION), 3 parts by weight of stearic acid, 5 parts by weight of zinc oxide, 2 parts by weight of 2,2,4-trimethyl-l,2-dihydroquinoline and 2 parts by weight of the condensation product of resorcin and acetone obtained in Example 1, Example 2, Example 3 or Example 4 are added into the mixer followed by kneading for 5 minutes at 50 rpm to obtain a rubber composition.

To the rubber composition obtained, 4 parts by weight of sulfur, 0.7 part by weight of N,N-dicyclohexyl-2-benzothiazylsulfenamide, 2 parts by weight of cobalt naphthenate (cobalt content: 10%) and 3 parts by weight of the methoxylated methylolmelamine resin (Sumikanol 507AP manufactured by Sumitomo Chemical Co., Ltd., content of the active components: 66% by weight) are added followed by conducting kneading using an open mill at a rubber temperature of 50 to 70°C to obtain an unvulcanized rubber composition. A vulcanized rubber composition is obtained by conduct vulcanization of the unvulcanized rubber composition obtained at 150°C for 25 minutes.

By using the condensation products of resorcin and acetone, rubber composition having better dispersibility of the condensation product than the rubber composition produced in the followed Comparative Example 2 can be obtained.

### Comparative Example 2

A vulcanized rubber composition is obtained according to the same manner as that described in Example 5 except that the condensation product of resorcin and acetone produced in Comparative Example 1 is used in place of the condensation product of resorcin and acetone produced in Example 1.

### Example 6

A belt is obtained by covering steel cords plated with brass with the unvulcanized rubber composition obtained in Example 5. An unvulcanized tire is molded by using the belt obtained according to a conventional process and the unvulcanized tire obtained is heated and pressurized in a vulcanizer to obtain a tire.

### Example 7

The extruding processing of the unvulcanized rubber composition obtained in Example 5 is conducted to prepare a rubber composition having a shape fitting on the carcass shape and it is applied up and down carcass fiber cord made of polyester to obtain a carcass. An unvulcanized tire is molded by using the carcass obtained according to a conventional process and the unvulcanized tire obtained is heated and pressurized in a vulcanizer to obtain a tire.

### Example 8

The extruding processing of the unvulcanized rubber composition obtained in Example 5 is conducted to obtain a captread. An unvulcanized tire is molded by using the captread obtained according to a conventional process and the unvulcanized tire obtained is heated and pressurized in a vulcanizer to obtain a tire.

### Example 9

The extruding processing of the unvulcanized rubber composition obtained in Example 5 is conducted to obtain a undertread. An unvulcanized tire is molded by using the undertread obtained according to a conventional process and the unvulcanized tire obtained is heated and pressurized in a vulcanizer to obtain a tire.

### Industrial Applicability

According to the present invention, a compound utilizable as a reinforcing agent capable of improving workability on the production of rubber products can be provided.

## Claims

1. A condensation product of resorcin and acetone, wherein the ratio of the area of the first elution peak to the total area of all peaks is 20 to 50% as determined by gel permeation chromatography of which detector is a differential refractive index detector.

2. The condensation product according to claim 1, wherein its melting point is 120°C or less.

3. A rubber composition comprising the condensation product according to claim 1 or 2, a rubber component, a filler and a sulfur component.

4. A belt comprising a steel cord covered with the rubber composition according to claim 3.

5. A carcass comprising a carcass fiber cord covered with the rubber composition according to claim 3.

6. A captread or undertread comprising the rubber composition according to claim 3.

7. A pneumatic tire produced by using the rubber composition according to claim 3.
